# EUROPÄISCHE PATENTANMELDUNG

(11) **EP 3 804 703 A1**
(43) Veröffentlichungstag der Anmeldung: **14.04.2021**
(21) Anmeldenummer: 19202461.0
(22) Anmeldetag: 10.10.2019
(51) Int. Cl.: A61K 9/51

(54) **VERFAHREN ZUR HERSTELLUNG EINES NANOPARTIKULÄREN WIRKSTOFFS**

(71) Anmelder: Bayer AG, 51373 Leverkusen (DE)
(72) Erfinder: Die Erfindernennung liegt noch nicht vor
(74) Vertreter: BIP Patents

(57) **Zusammenfassung**

Die vorliegende Erfindung betrifft ein Verfahren zur Herstellung eines nanopartikulären Wirkstoffs, umfassend die Schritte: a) Bereitstellen einer Lösung eines Wirkstoffs in einem Lösungsmittel, Bereitstellen eines Anti-Lösungsmittels für den Wirkstoff und Bereitstellen eines Stabilisators, wobei der Stabilisator in dem Lösungsmittel und/oder dem Anti-Lösungsmittel gelöst vorliegt und b) Mischen der Lösung des Wirkstoffs in dem Lösungsmittel, des Antilösungsmittels und des Stabilisators in einem Mikromischer unter Erhalt einer Suspension umfassend ausgefällten Wirkstoff, das Lösungsmittel und das Anti-Lösungsmittel, wobei der ausgefällte Wirkstoff in Form von Nanoteilchen vorliegt. Nach Schritt b) wird der Schritt c) durchgeführt: c) Verdampfen des Lösungsmittels und des Anti-Lösungsmittels unter Erhalt von Aggregaten des nanopartikulären Wirkstoffs.

## Beschreibung

Die vorliegende Erfindung betrifft ein Verfahren zur Herstellung eines nanopartikulären Wirkstoffs, umfassend die Schritte: a) Bereitstellen einer Lösung eines Wirkstoffs in einem Lösungsmittel, Bereitstellen eines Anti-Lösungsmittels für den Wirkstoff und Bereitstellen eines Stabilisators, wobei der Stabilisator in dem Lösungsmittel und/oder dem Anti-Lösungsmittel gelöst vorliegt und b) Mischen der Lösung des Wirkstoffs in dem Lösungsmittel, des Antilösungsmittels und des Stabilisators in einem Mikromischer unter Erhalt einer Suspension umfassend ausgefällten Wirkstoff, das Lösungsmittel und das Anti-Lösungsmittel, wobei der ausgefällte Wirkstoff in Form von Nanoteilchen vorliegt.

Ein hoher Anteil pharmazeutischer Wirkstoffe von über 40 % sind der BCS (Biopharmaceutics Classification System) Klasse 2 bzw. 4 zuzuordnen. Die Wirkstoffe in diesen Klasse verfügen über eine schlechte Löslichkeit und hohe Bioverfügbarkeit bzw. schlechte Löslichkeit und schlechte Bioverfügbarkeit. Um die schlechte Löslichkeit zu verbessern ist es eine allgemein akzeptierte Methode die Partikelgröße der Wirkstoffe zu reduzieren und so ein großes Oberflächen-zu-Volumenverhältnis zu schaffen. Entsprechend der Noyes-Whitney-Gleichung wird so eine hohe Löslichkeit der Partikelgrenzschicht geschaffen.

Bekannte Top-down Methoden wie zum Beispiel die Nanomahlung oder die Hochdruckhomogenisation werden standardmäßig zur Herstellung von Mikro- und Nano-Wirkstoffpartikeln genutzt. Jedoch weisen diese Methoden einige Limitierungen auf, wie zum Beispiel ein hoher Energieeintrag, geringe Ausbeuten, Kontamination durch Abrieb, und schwer kontrollierbare Partikelgrößen und Oberflächeneigenschaften, welche ihre Anwendung und weitere Kommerzialisierung erschwert.

Der, dieser Erfindung zu Grunde liegende Fällungsprozess (Bottom-up Methode) ist ein dem Fachmann bekannte Standardmethode um Mikro- und Nanopartikeln herzustellen. Entsprechend der Vorarbeiten anderer Wissenschaftler ist bekannt, dass ein Reaktor mit exzellenter Mischqualität entscheidend für die gleichmäßige Herstellung von Wirkstoff-Nanopartikeln ist. Ein Mikrokanalreaktor bietet ein definiertes Reaktionsvolumen und einen definierten Kanal, welcher in Hinsicht auf Konzentration (Stofftransport) und Temperatur (Wärmetransport) zu einer besseren Reaktionskontrolle führt. Der Fällungsschritt selbst wird im Mikrokanalreaktor durch eine schnelle Vermischung, die zu einer schnellen Übersättigung führt, kontrolliert. Die Übersättigung wiederum bestimmt die Partikelgröße und ihre Verteilung, wie zum Beispiel Keimbildungsrate und Keimwachstum.

Die Kombination aus Fällung und Mikrokanalreaktor um Mikro- und Nanopartikeln herzustellen wurden in zahlreichen Publikationen und Patenten beschrieben. Die Partikelgrößen bewegen sich dabei in Bereichen zwischen 100 nm und 80 µm.

US 2013/0012551 A1 beschreibt eine Methode zur Herstellung von Mikro- und Nanopartikeln aus wasserlöslichen und wasser-unlöslichen Substanzen durch die Fällung in einem Mikrojet Reaktor. Dabei werden Solvent, enthaltend das zu fällende Produkt und ein Antisolvent, welche in Form von Jets in einem Mikrojet-Reaktor bei definierten Drücken und Volumenströmen gemischt, um eine schnelle Fällung, Co-Fällung oder eine chemische Reaktion zu beeinflussen, bei der Mikro- oder Nanopartikeln entstehen. Die Partikelgröße wird dabei über die Temperatur im System, den Volumenströmen des Solvent und Antisolvent und/oder des Gases kontrolliert. Kleinere Partikelgrößen werden dabei bei niedrigen Temperaturen, bei hohen Solvent und Antisolvent Volumenströmen und/oder vollständigen Abwesenheit des Gasstroms erzielt. In der Methode wird nur ein Mikrojet Reaktor zur Fällung genutzt. Die Partikelgrößen liegen im Bereich zwischen 141,2-358 nm. Nachteilig ist bei dieser Methode, dass die Partikelgrößenstabilität, insbesondere die Langzeitstabilität nicht behandelt wird.

Hong Zhao et al., Ind. Eng. Chem. Res. 2007, 46, 8229 - 8235 beschreibt eine Solvent Antisolvent Fällung (LSAP) in einem Mikrokanalreaktor. Die Partikelgröße wurde dabei von 55 µm zu 364 nm reduziert. Nachteilig bei dieser Methode ist die Filtrierung der Probe nach der Fällung mit einem 0,45 µm Filter. Nachteilig ist bei dieser Methode, dass die Partikelgrößenstabilität, insbesondere die Langzeitstabilität nicht behandelt wird.

Yuancai Dong et al., Powder Technology 2014, 268, 424-428, kombinieren Antisolvent-Fällung in einem Mikrokanalreaktor mit Sprühtrocknung. Es werden Partikelgrößen zwischen 196-296 nm erzielt. Die Gesamtvolumenströme liegen maximal bei 4 ml/min. Nachteilig ist, dass der Prozess für den Industriellen Scale-Up nicht geeignet ist. Nachteilig ist auch, dass obwohl die Redispergierung in einer Wasser-SDS Lösung erfolgte, keine nahezu vollständige Redispergierung möglich ist. Die redispergierte Suspension weist mittlere Partikelgrößen um die 350 nm auf.

Jiahui Hu et al European Journal of Pharmaceutical Sciences 2003, 20, 295-303, entwickelten die Sprühgefrierung in Flüssigkeiten (SFL Technologie) um mikronisierte Pulver zu erhalten. Dabei wird eine Wirkstofflösung direkt in eine kryogenen Flüssigkeit eingesprüht um gefrorene nanostrukturierte Partikel zu erhalten. Diese werden anschließend mittels Gefriertrocknung getrocknet. Dabei werden bei der Partikelgrößenverteilung D(10)-Werte bei 0,14 µm, D(50)-Werte bei 0,68 µm und D(90)-Werte bei 15,89 µm erzielt. Nachteilig ist, dass bei diesem Prozess die Polydispersität der hergestellten Partikel sehr breit ist. Zudem müssen kritische Lösemittel wie Acetonitrile oder THF zur Lösung des Wirkstoffs verwendet. Der Wirkstoffgehalt in der Ursprungslösung liegt bei maximal 2,2 wt.-%.

Niva et al (2013) kombinieren die Nassmahlung mit der Sprüh-Gefriertrocknung. Als Stabilisatoren werden PVP (Polymer) und SLS (Tensid) genutzt. Die Partikelgrößen nach der Mahlung liegen zwischen 170-180 nm. Nachteilig ist, dass die redispergierten Partikel bei 1-100 µm liegen.

Mahesh V. Chaubal Pharmaceutical Research, 2008, 25, 10, berichtet von redispergierbaren Nanopartikelenthaltenden Pulvern und der Wichtigkeit von geladenen Tensiden in Bezug auf die Stabilität von Partikeln während der Trocknung. Nachteilig an dieser Methode ist, dass die durchschnittliche Partikelgröße der Suspensionen nach der Redispergierung stets 10-20% größer ist als im Vergleich zu den Ursprungssuspensionen vor der Trocknung. Des Weiteren werden zusätzlich zu den Stabilisierungsadditiven (Poloxamer 188 und Natriumdesoxycholat) Gerüstbildner wie Lactose, Saccharose und Mannitol verwendet. Zusätzlich sind die Partikeln wesentlich gröber (99% < 1µm) (auch vor der Trocknung (99%<0,8µm).

Andrej Dolenc International Journal of Pharmaceutics, 2009, 376, 204-212 et al, stellt Nano-Suspensionen mit PVP und SDS als Additive her und trocknet diese mittels Sprühtrocknung. Nachteilig an dieser Methode ist, dass die Pulver zwar redispergierbar sind, allerdings sind deutliche Abweichungen im D(90)-Wert (30%) zwischen der Ursprungs- und redispergierten Suspension zu erkennen. Die möglichen Vorteile von ionischen Tensiden für die Stabilität der Nanopartikeln während der Trocknung sind nicht beschrieben. SDS fungiert nur als Additiv für die Bildung stabiler Nanopartikel mittels Fällung. Zusätzlich liegen wesentlich gröbere Partikeln im D(90)-Bereich vor (>1µm).

Die vorliegende Erfindung hat sich die Aufgabe gestellt, ein Verfahren zur Herstellung von gut redispiergierbaren Nanopartikeln bereitzustellen.

Erfindungsgemäß gelöst wird diese Aufgabe durch ein Verfahren gemäß Anspruch 1. Vorteilhafte Weiterbildungen sind in den Unteransprüchen angegeben. Sie können beliebig kombiniert werden, sofern sich aus dem Kontext nicht eindeutig das Gegenteil ergibt.

Ein Verfahren zur Herstellung eines nanopartikulären Wirkstoffs umfasst die Schritte:
a) Bereitstellen einer Lösung eines Wirkstoffs in einem Lösungsmittel, Bereitstellen eines Anti-Lösungsmittels für den Wirkstoff und Bereitstellen eines Stabilisators, wobei der Stabilisator in dem Lösungsmittel und/oder dem Anti-Lösungsmittel gelöst vorliegt und
b) Mischen der Lösung des Wirkstoffs in dem Lösungsmittel, des Antilösungsmittels und des Stabilisators in einem Mikromischer unter Erhalt einer Suspension umfassend ausgefällten Wirkstoff, das Lösungsmittel und das Anti-Lösungsmittel,
wobei der ausgefällte Wirkstoff in Form von Nanoteilchen vorliegt,
wobei nach Schritt b) der Schritt c) durchgeführt wird:
c) Verdampfen des Lösungsmittels und des Anti-Lösungsmittels unter Erhalt von Aggregaten des nanopartikulären Wirkstoffs.

Ein Merkmal mikrostrukturierter Bauteile wie Mikromischern sind die kleinen Abmessungen der Fluidkanäle, die typischerweise im Bereich zwischen 10 und 5000 um angesiedelt sind. Aus diesem Grund können beispielsweise mit Multilaminationsmischern feine Fluidlamellen erzeugt werden, zwischen denen aufgrund ihrer geringen Dicke ein schneller Stoffaustausch durch Diffusion erfolgen kann. Vorzugsweise enthalten die Mikromischer Mischplatten mir Nenndurchmessern der Schlitze zwischen 100 und 400 µm.

Als Wirkstoff kommt grundsätzlich jede organische Verbindung in Frage, die in der Lage ist, in einem biologischen System eine vorbestimmte und gewünschte Wirkung zu erzielen. Beispielsweise kann es sich um pharmazeutische Wirkstoffe oder agrochemische Wirkstoffe handeln. Vorzugsweise handelt es sich bei den Wirkstoffen um Verbindungen, die in Wasser eine Löslichkeit von ≤ 10 mg/ml bei 20 °C aufweisen.

Geeignete Wirkstoff-Kandidaten können aus einer Vielfalt von bekannten Wirkstoffklassen ausgewählt sein, zum Beispiel aus Schmerzmitteln, Antiinflammatorika, Anthelminthika, Antiarrhythmika, Antibiotika (einschließlich Penicillinsäuren), Antikoagulantien, Antideppressiva, Antidiabetika, Antiepileptika, Antihistaminika, Antihypertonika, Anticholinergika, Antimycobakterika, Antineoplastika, Cytostatika, Immunosuppressiva, Thyreostatica, Blutprodukte und Ersatzstoffe, Inotropika, Kontrastmittel, Corticosteroide, Beruhigungsmittel gegen Husten (Expektoranzien und Mukolytika), Diagnostika, bildgebende Diagnostika, Diuretika, Dopaminerg (Antiparkinson Wirkstoffe), Hämostatika, Immunologische Wirkstoffe, Lipid regulierende Wirkstoffe, Muskelrelaxantia, Parasympathomimetika, Kalzitonine und Bisphosphonate, Prostaglandine, Radio-Pharmazeutika, Sexual-Hormone (einschließlich Steroide), Anti-Allergika, Stimulanten und Anoretika, Sympathomimetika, Thyreoidea-Wirkstoffe, Vasodilatatoren und Xanthine, bevorzugt Wirkstoffe, die oral und parenteral Anwendung finden.

Vorzugsweise ist der pharmazeutische Wirkstoff ausgewählt aus: Alminoprofen, Benoxaprofen, Bucloxic Acid, Carprofen, Fenbufen, Fenoprofen, Fluprofen, Flurbiprofen, Ibuprofen, Indoprofen, Ketoprofen, Miroprofen, Naproxen, Oxaprozin, Pirprofen, Pranoprofen, Suprofen, Tiaprofenic Acid, Tioxaprofen, Indomethacin, Acemetacin, Alclofenac, Clidanac, Diclofenac, Fenclofenac, Fenclozic Acid, Fentiazac, Furofenac, Ibufenac, Isoxepac, Oxpinac, Sulindac, Tiopinac, Tolmetin, Zidometacin, Zomepirac, Flufenamic Acid, Meclofenamic Acid, Mefenamic Acid, Niflumic Acid, Tolfenamic Acid, Diflunisal, Flufenisal, Isoxicam, Piroxicam, Sudoxicam, Tenoxican, Acetyl Salicylic Acid, Sulfasalazine, Apazone, Bezpiperylon, Feprazone, Mofebutazone, Oxyphenbutazone, Phenylbutazone, Vericiguat, Riociguat oder einer Mischung aus mindestens zwei der vorgenannten Wirkstoffe. Besonders bevorzugt sind Indomethacin, Naproxen und Vericiguat.

Im erfindungsgemäßen Verfahren können Nano-Wirkstoffpartikel mittels kontinuierlicher Fällungsmethode über eine Mikroreaktionstechnologie hergestellt werden. Die zu fällende Komponente ist dabei in einem Lösemittel (nachfolgend auch "Solvent" genannt), in dem es gut löslich ist, gelöst. Ein weiteres Lösemittel, welches bevorzugt vollständig mit dem Solvent mischbar ist, wird mit dem Solvent vermischt. Dieses wird als Anti-Lösungsmittel oder AntiSolvent bezeichnet. Die zu fällende Komponente ist dabei schwer löslich im Antisolvent und wird durch lokale Übersättigung zur Keimbildung getrieben. Die Keime wachsen zu Partikeln an.

Das Lösungsmittel kann ein beliebiges organisches Lösemittel sein, welches den Wirkstoff adäquat löst. Das Lösemittel sollte mit dem Antisolvent mischbar sein. Bevorzugt weist das ausgewählte Solvent ideales Mischverhalten mit dem Antisolvent auf, sodass die Lösung verzögerungsfrei in der Partikelsuspension verteilt wird. Geeignete organische Lösemittel schließen ein, aber sind nicht limitiert auf Methanol, Ethanol, Isopropanol, 1-Butanol, Trifluoroethanol, Tetrahydrofuran, Propionaldehyd, Aceton, N-Propylamin, Isopropylamin, Ethylendiamin, Acetonitril, Methylethylketon, Essigsäure, Ameisensäure, Dimethylsulfoxid, Diemthylformamid, N-Methyl-2-pyrrolidon 1,3-Dioxolan, Hexafluoroisopropanol und Kombinationen davon.

Das Anti-Lösemittel ist eine Flüssigkeit, in der der Wirkstoff schwerlöslich (Löslichkeitskonzentration ≤ 10 mg/ml) ist. Wasser ist das bevorzugte Antisolvent. Der Volumenstrom des Antisolvents kann vom Volumenstrom des Solvents oder vom Verhältnis des Antisolvent zu Solvent abhängen, aber ist typischerweise im Bereich von 1:2 bis 200:1 (bevorzugt 1:1 bis 100:1, besonders bevorzugt 1:1 bis 1:20).

Es ist möglich, dass in Schritt a) ein Tensid im Antilösungsmittel zugegeben wird, um eine verbesserte Redispergierbarkeit nach der Trocknung ohne Ultraschallbehandlung zu erreichen.

Zur Unterbindung des Partikelwachstums in Lösung und der Stabilisierung der Partikelgröße wird der Trocknungsschritt c) vorzugsweise direkt nach der Fällung durchgeführt. Die Trocknung kann auch eine verbesserte Handhabung bezüglich Schütt- und Fließfähigkeit der wirkstoffpartikelenthaltenden Feststoffe für die Weiterverarbeitung in oralen und parenteralen Darreichungsformen bieten. Geeignete Verfahren sind unter anderem die Gefriertrocknung, Sprühtrockung und die Sprüh-Gefriertrocknung. Dadurch entstehen fließfähige Feststoffe.

Die nach Schritt c) erhaltenen Aggregate des nanopartikulären Wirkstoffs können ganz oder teilweise auch als Agreggate des nanopartikulären Wirkstoffs eingebettet in einer Matrix des Stabilisators vorliegen.

Zur Herstellung einer galenischen Darreichungsform können die nach Schritt c) erhaltenen Aggregate des nanopartikulären Wirkstoffs wieder in einem Anti-Lösungsmittel redispergiert werden. Zur Beeinflussung des Redispergierverhaltens können Kombinationen aus Polymeren und ionischen Tensiden in verschiedenen Konzentrationsverhältnissen eingesetzt werden, um die Partikelgrößenverteilung (PGV) der Ursprungssuspension zu erhalten.

Bevorzugt ist wenn die mittlere Partikelgröße (bestimmt mittels Laserstreuung gemäß ISO 13320) um nicht mehr als 500 % von der ursprünglichen mittleren Partikelgröße der gefällten Nanosuspension abweicht, bevorzugt < 100 %, und besonders bevorzugt < 50 %, ganz besonders bevorzugt < 10 %. Insbesondere wird eine Langzeitstabilität der redispergierten Nanopartikelsuspension im erfindungsgemäßen Verfahren ermöglicht. Bevorzugt liegt der D(90)-Wert der Partikelgrößenverteilung für die redispergierten Teilchen, bestimmt mittels dynamischer Lichtstreuung, unterhalb von 700 nm, weiter bevorzugt unterhalb von 400 nm und noch weiter bevorzugt unterhalb von 300 nm.

Die anderen Patentansprüche haben im direkten Vergleich zur vorliegenden Literatur und anderen Patenten sonst keine besonderen Vorteile.

Mikromischer im erfindungsgemäßen Verfahren sind Mischer zum Vermischen von mindestens zwei Fluidströmen, in denen interne Leitungen Durchmesser von weniger als einem Millimeter aufweisen. Ein- oder mehr Zentrifugalpumpen, ein in-line Homogenisator, ein Ultraschallmischer, ein Mikromischer und weitere Kombinationen von solchen Mischern können auch verwendet werden, im Besonderen in den Fällen, bei denen es erwünscht ist, die Verweilzeit in der Mischzone zu erhöhen. Bevorzugte Mischer sind Mikrokanalreaktoren wie Ventilmikromischer, Kaskadenmikromischern, LH Typ Mikromischern, etc. Diese Mikrokanalreaktoren bieten verbesserte Mikrovermischungseffekte und dadurch eine enge Partikelgrößenverteilung und kleinere Partikelgrößen.

In einer besonders bevorzugten Ausführungsform dient ein Kaskadenmischer mit Injektionsmodul einer homogenen Vermischung des Solvent- und Antisolvent-Stroms.

Das erfindungsgemäße Verfahren wird vorzugsweise als kontinuierliches Verfahren durchgeführt. Dann hat es den Vorteil eines einstufigen kontinuierlichen Prozesses basierend auf dem homogenen Keimbildungsmechanismus. Dieser liefert in einen effizienten Prozess ein gleichmäßigeres Produkt und eine kleinere Partikelgröße.

Das erfindungsgemäße Verfahren kann mit kurzen Verweilzeiten im Mikromischer durchgeführt werden ohne weiteren Energieeintrag und bei Atmosphärendruck. Die Verweilzeiten können beispielsweise im Bereich von ≥ 0.01 Sekunden bis ≤ 0.4 Sekunden liegen.

Das Gewichtsverhältnis von Wirkstoff im gesamten Feststoffanteil nach der Redispergierung in Wasser kann bei bis zu 70 % liegen.

Die Wirkstoffkonzentration in der Lösung ist bevorzugt nahe der praktischen Löslichkeitsgrenze des Lösemittels. Solche Konzentrationen hängen vom ausgewählten Wirkstoff und Lösemittel ab, aber sind typischerweise im Bereich von 0.1 bis 20 Gewichts-%.

Bei dem Stabilisator handelt es sich um ein oder mehrere Additive, welche das Partikelwachstum hemmen, die Aggregation der Nanopartikel unterdrücken oder als Gerüstbildner die Redispergierbarkeit der Nanopartikel verbessern. Die Zugabe des Stabilisators kann bevorzugt im Solvent oder im Antisolvent erfolgen. Die Wahl des Stabilisators oder der Stabilisatoren sind vom Wirkstoff-Molekül abhängig, sowie aber auch von der Stabilisator-Solvent bzw. Stabilisator-Antisolvent-Wechselwirkung. Stabilisatoren schließen Polymere, Copolymere, Polyelektrolyte, Metallsalze und ionische und nicht-ionische Tenside ein.

Die Konzentration der Stabilisatoren hängt von der Wirkstoffkonzentration oder dem Gewichtsverhältnis von Wirkstoff zu Stabilisator in der Suspension ab. Letzteres bewegt sich typischerweise im Bereich von 1:100 bis 100:1 (bevorzugt 1:10 bis 100:1, besonders bevorzugt 1:1 bis 100:1).

Die resultierenden Nanopartikel, welche in der Nanosuspension vorhanden sind, haben vorzugsweise eine durchschnittliche Partikelgröße, ohne Filitration, von ≥ 10 nm bis ≤ 999 nm (bestimmt mittels Laserstreuung gemäß ISO 13320).

In einer Ausführungsform des Verfahrens beinhaltet Schritt c) das Bereitstellen von Tröpfchen der Suspension und die Tröpfchen in Schritt c) weisen direkt nach ihrer Bereitstellung einen Durchmesser von ≥ 0.001 mm bis ≤ 3 mm auf. Bevorzugt ist ein Tröpfchendurchmesser von ≥ 0,001 mm bis ≤ 0,12 mm, mehr bevorzugt ≥ 0,001 mm bis ≤ 0,03 mm.

In einer weiteren Ausführungsform des Verfahrens weisen die in Schritt b) ausgefallenen Nanoteilchen eine durchschnittliche Teilchengröße (bestimmt mittels Laserstreuung gemäß ISO 13320) von ≥ 20 bis ≤ 300 nm auf. Vorzugsweise beträgt diese Größe ≥ 50 nm bis ≤ 200 nm.

In einer weiteren Ausführungsform des Verfahrens ist Schritt c) ein Sprühtrocknungsschritt, ein Sprüh-Gefriertrocknungsschritt oder ein Gefriertrocknungsschritt.

In einer weiteren Ausführungsform des Verfahrens weisen die erhaltenen Aggregate des nanopartikulären Wirkstoffs einen maximalen Durchmesser von ≥ 0.001 mm bis ≤ 1 mm auf. Bevorzugt ist ein Durchmesser von ≥ 0.001 mm bis ≤ 0,1 mm, mehr bevorzugt ≥ 0.001 mm bis ≤ 0,025 mm.

In einer weiteren Ausführungsform des Verfahrens liegen der Volumenstrom des Solvents mit gelöstem Wirkstoff und der Volumenstrom des Antisolvents in einem Volumenverhältnis von Lösungsmittel zu Anti-Lösungsmittel ≥ 1:100 bis ≤ 1:1 zueinander. Vorzugsweise beträgt das Volumenverhältnis ≥ 1: ≤ 40, mehr bevorzugt ≥ 1 : ≤ 20, weiter bevorzugt ≥ 1 : ≤ 10, noch weiter bevorzugt ≥ 1 : ≤ 5 und besonders bevorzugt ≥ 1 : ≤ 1.

In einer weiteren Ausführungsform des Verfahrens weisen das Lösungsmittel und das Anti-Lösungsmittel einen messbaren pH-Wert auf und der pH-Wert des Lösungsmittels unterscheidet sich von dem pH-Wert des Anti-Lösungsmittels. Somit kann durch eine Veränderung des pH-Wertes ("pH-Shift") beim Mischen ein Ausfällen des Wirkstoffs erreicht werden. Der pH-Wert kann insbesondere durch eine pH-Einstabmesskette mit einer Silber/Silberchloridelektrode als Referenz bestimmt werden. In der Regel enthalten zur aussagekräftigen Bestimmung des pH-Werts das Lösungsmittel und das Anti-Lösungsmittel Wasser. Vorzugsweise handelt es sich gemäß dieser Ausführungsform bei dem Lösungsmittel und Anti-Lösungsmittel durch Säure- und/oder Basenzugabe auf die gewünschten pH-Werte eingestelltes Wasser. Der Unterschied der pH-Werte beträgt vorzugsweise ≥ 1, mehr bevorzugt ≥ 2 und besonders bevorzugt ≥ 3.

In einer weiteren Ausführungsform des Verfahrens liegt der Stabilisator im Lösungsmittel und/oder im Anti-Lösungsmittel gelöst vor.

In einer weiteren Ausführungsform des Verfahrens ist der Mikromischer ein Ventilmischer oder ein Kaskadenmischer. In einem Ventilmischer kann eine Rückströmsperre das Zurückströmen der Mischung in Zuführleitungen fast vollständig oder vollständig verhindern. Bevorzugte Ventilmikromischer sind solche mit einem ersten Kanal für die Zufuhr eines ersten Teilstroms und mit einem zweiten Kanal für die Zufuhr eines zweiten Teilstroms, die in flachen Eintrittsspalten in eine Misch-und Reaktionszone münden und die Misch- und Reaktionszone über einen Auslasskanal verlassen, wobei zwischen der Misch-und Reaktionszone und mindestens einem Kanal für die Zufuhr eines Teilstroms eine Rückströmsperre angeordnet ist. Einer der Zufuhrkanäle weist vorteilhafterweise eine Rückströmsperre in dem Abschnitt auf, in dem der Zufuhrkanal sich zur Misch- und Reaktionszone aufweitet. Solche Mikromischer sind unter anderem in WO 2005/079964 A1 beschrieben.

Das Mischprinzip von Kasadenmischern beruht auf der sogenannten split-and-recombine-Operation. Bevorzugt ist hier ein statischer Mikrovermischer mit Zuführkammern für mindestens zwei zu vermischende Fluide, von denen Mikrokanäle zu einer Mischkammer führen, wobei die Mikrokanäle in mindestens zwei aneinanderliegenden Zuführelementen angeordnet sind, wobei die Zuführelemente keilförmige Platten sind, die mindestens zu einem Ringsektor zusammensetzbar sind, der die Mischkammer bogenförmig umgibt, und wobei die für jedes Fluid vorgesehenen Mikrokanäle eine symmetrische, mindestens zwei Stufen umfassende Bifurkationskaskade bilden. Solche Mikromischer sind unter anderem in WO 2001/043857 A1 beschrieben.

In einer weiteren Ausführungsform des Verfahrens wird nach Schritt b) die Suspension vom Mischer direkt in eine Sprühdüse eingeleitet und dort in Schritt c) versprüht und getrocknet.

In einer weiteren Ausführungsform des Verfahrens umfasst der Stabilisator einen Zucker, eine Aminosäure oder eine Mischung hieraus. Hierbei handelt es sich um sogenannte Gerüstbildner. Geeignet sind zum Beispiel Mannitol, Lactose, Glycine, Trehalose, Sucrose, Cyclodextrine oder Mischungen aus mindestens zwei der vorgenannten Substanzen. Gerüstbildner verbessern die Abfüllbarkeit und Prozessierbarkeit des getrockneten Materials.

In einer weiteren Ausführungsform des Verfahrens umfasst der Stabilisator ein ionisches Tensid. Das ionische Tensid kann ein anionisches, kationisches oder zwitterionisches (amphoteres) Tensid sein. Ohne auf eine Theorie festgelegt zu sein wird angenommen, dass das ionische Tensid sich insbesondere in Kombination mit einem Polymer positiv auf die Stabilität der Wirkstoffpartikel während der Trocknung auswirkt. Die Partikel sind folglich durch die Kombination aus elektrostatischer und sterischer Stabilisierung leichter redispergierbar. Zudem kann beobachtet werden, dass ein polymorpher Zustand der Partikel erhalten bleibt. Dieses lässt sich sowohl mittels Röntgenpulverdiffraktometrie als auch mit Fourier-transformierter Infrarot-Spektroskopie dokumentieren.

In den Polymer/Wirkstoffpartikeln in Schritt b) kann der Polymergehalt ≥ 0.05 bis ≤ 3 Gewichts-% und der Tensidgehalt ≥ 0.05 bis ≤ 0,1 Gewichts-%, jeweils bezogen auf das Gesamtgewicht der Suspension, betragen. Ein weiteres Beispiel für eine Dosierung ist ein Gewichtsverhältnis von Wirkstoff: Polymer : Tensid von ≥ 0.1 bis ≤ 5 : 1 : ≥ 0.024 bis ≤ 2.

In einer weiteren Ausführungsform ist das ionische Tensid ausgewählt aus:
Acylaminosäuren (und deren Salze), wie: Acylglutamate, beispielsweise Natriumacylglutamat, Di-TEA-palmitoylaspartat und Natriucaprylglutamat; Acylpeptide, beispielsweise Palmitoylhydrolysiertes Milchprotein, Natrium Cocoylhydrolysiertes Soja Protein und Natrium-/Kalium Cocoyl-hydrolysiertes Kollagen; Sarcosinate, beispielsweise Myristoyl Sarcosin, TEA-lauroyl Sarcosinat, Natriumlauroylsarcosinat und Natriumcocoylsarkosinat; Taurate, beispielsweise Natriumlauroyltaurat und Natriummethylcocoyltaurat; Acyllactylate, Luroyllactylat, Caproyllactylat, Alaninate; Carbonsäuren und Derivate, wie: Carbonsäuren, beispielsweise Laurinsäure, Aluminiumstearat, Magnesiumalkanolat und Zinkundecylenat, Ester-Carbonsäuren, beispielsweise Calciumstearoyllactylat und Natrium PEG-Lauramidcarboxylat, EtherCarbonsäuren, beispielsweise Natriumlaureth-Carboxylat und Natrium PEG-Cocamide Carboxylat; Phosphorsäureester und Salze, wie beispielsweise DEA-Oleth--Phosphat und Dilaureth-Phosphat; Sulfonsäuren und Salze, wie Acyl-isethionate, z.B. Natrium-/ Ammoniumcocoyl-isethionat, Alkylarylsulfonate, Alkylsulfonate, beispielsweise Natriumcocosmonoglyceridsulfat, Natrium C-Olefin-sulfonat, Natriumlaurylsulfoacetat und Magnesium PEG-Cocamidsulfat, Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat; sowie Schwefelsäureester, wie Alkylethersulfat, beispielsweise Natrium-, Ammonium-, Magnesium-, MIPA-, TIPA-Laurethsulfat, Natriummyrethsulfat und Natrium C-Parethsulfat, Alkylsulfate, beispielsweise Natrium-, Ammonium-und TEA-Laurylsulfat.

Erfindungsgemäß können das oder die ionischen Tenside ferner vorteilhaft gewählt werden aus der Gruppe der kationischen Tenside. Vorteilhaft zu verwendende kationische Tenside sind Alkylamine, Alkylimidazole, ethoxylierte Amine, quaternäre Tenside und Esterquats

Quaternäre Tenside enthalten mindestens ein N-Atom, das mit 4 Alkyl- oder Arylgruppen kovalent verbunden ist. Dies führt, unabhängig vom pH Wert, zu einer positiven Ladung. Vorteilhaft sind, Alkylbetain, Alkylamidopropylbetain und Alkyl-amidopropylhydroxysulfain. Die erfindungsgemäß verwendeten kationischen Tenside können ferner bevorzugt gewählt werden aus der Gruppe der quaternären Ammoniumverbindungen, insbesondere Benzyltrialkylammoniumchloride oder - bromide, wie beispielsweise Benzyldimethylstearylammoniumchlorid, ferner Alkyltrialkylammoniumsalze, beispielsweise beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersultate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

Erfindungsgemäß können das oder die ionischen Tenside vorteilhaft gewählt werden aus der Gruppe der amphoteren Tenside.

Vorteilhaft zu verwendende amphotere Tenside sind: Acyl-/dialkylethylendiamine, beispielsweise Natriumacylamphoacetat, Dinatriumacylamphodipropionat, Dinatriumalkylamphodiacetat, Natriumacylamphohydroxypropylsultonat, Dinatriumacylamphodiacetat und Natriumacylamphopropionat sowie N-Alkylaminosäuren, beispielsweise Aminopropylalkylglutamid, Alkylaminopropionsäure, Natuiumalkylimidodipropionat und Lauroamphocarboxyglycinat.

Besonders bevorzugt als Tensid ist Natriumdodecylsulfat (SDS), Natriumdocusat, Natriumoleat und/oder Natriumdesoxycholat.

In einer weiteren Ausführungsform des Verfahrens umfasst der Stabilisator ein wasserlösliches Polymer. Unter "wasserlöslich" ist hierbei zu verstehen, dass sich bei 20 °C in 100 g Wasser mindestens 0.5 g, bevorzugt mindestens 2 g des Polymers auflösen beziehungsweise unter Gelbildung lösen.

Das Polymer kann ausgewählt sein aus der Gruppe: Alkylcellulosen, Hydroxyalkylcellulosen, Hydroxyalkylalkylcellulosen, Carboxyalkylcellulosen, Alkalimetallsalze der Carboxyalkylcellulosen, Carboxyalkylalkylcellulosen, Carboxyalkylcelluloseester, Stärken, Pektine, Chitinderivate, Polysaccharide, Polyacrylsäure und ihre Salze, Polymethacrylsäure und ihre Salze, Polyvinylalkohol, Polyvinylpyrrolidon, Polyalkylenoxide oder eine Mischung aus mindestens zwei der vorgenannten Polymere.

Vorzugsweise ist das Polymer ausgewählt aus: Methylcellulose, Hydroxymethylcellulose, Hydroxyethylcellulose, Hydroxypropylcellulose, Hydroxybutylcellulose, Hydroxyethylmethylcellulose, Hydroxypropylmethylcellulose, Carboxymethylcellulose, Natriumcarboxymethylcellulose, Carboxymethylethylcellulose, Carboxyalkylcelluloseester, Stärken, Natriumcarboxymethylamylopektin, Chitosan, Dextransulfat Natriumsalz, Alginsäure, Alkalimetall- und Ammoniumsalze der Alginsäure, Carrageenane, Galaktomannane, Traganth, Agar-Agar, Gummi arabicum, Guargummi, Xanthangummi, Polyacrylsäure und ihre Salze, Polymethacrylsäure und ihre Salze, Polyvinylalkohol, Polyvinylpyrrolidon, Polyethylenoxid, Polypropylenoxid, Copolymere aus Ethylenoxid und Propylenoxid, N-Vinylpyrrolidon-VinylacetatCopolymere oder eine Mischung aus mindestens zwei der vorgenannten Polymere. Besonders bevorzugt sind Polyvinylpyrrolidone (insbesondere K12 und K30-Typen) und N-Vinylpyrrolidon-Vinylacetat-Copolymere.

Vorteilhaft sind weiterhin Stabilisatoren, welche ein wasserlösliches Polymer und ein ionisches Tensid umfassen.

In einer weiteren Ausführungsform des Verfahrens ist das Lösungsmittel ein Alkanol. Beispiele sind Methanol, Ethanol, Isopropanol, n-Propanol, THF, DMSO, DMF, NMP und Mischungen aus wenigstens zwei der vorgenannten Substanzen. In einer weiteren Ausführungsform des Verfahrens ist das Anti-Lösungsmittel Wasser.

Die vorliegende Erfindung wird anhand der nachfolgenden Figuren und Beispiele näher erläutert, ohne jedoch darauf beschränkt zu sein.

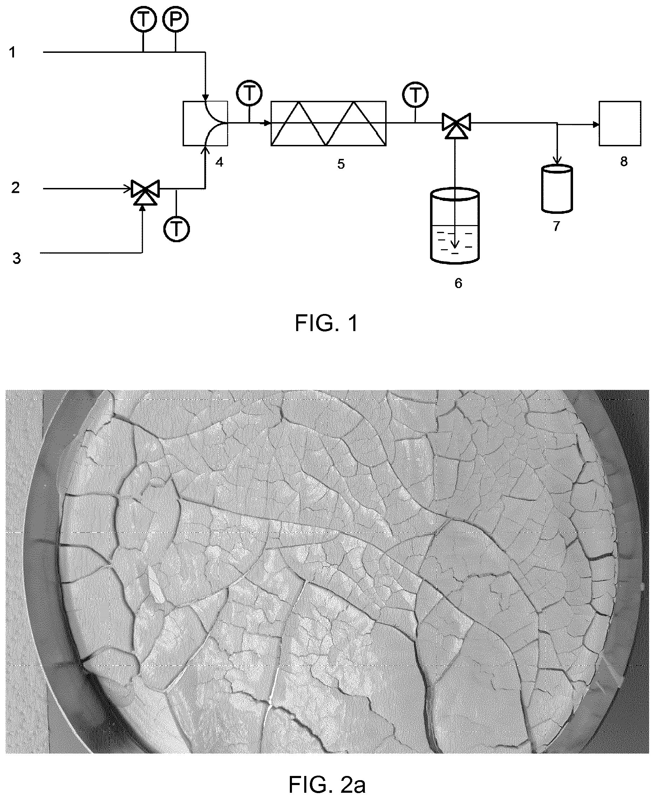

FIG. 1 ist eine schematische Darstellung des Prozesses in der vorliegenden Erfindung. Der Prozess beinhaltet den Solvent Feed 1 und Antisolvent Feed 2, welche im Mikromischer 4 ideal vermischt werden, danach strömt die Mischung in den Mikrokanalreaktor 5, in der die Keimbildung und Wachstum zu Mikro- oder Nanopartikel stattfindet. Die Partikelsuspensionen werden in den Probenbehälter 7 aufgefangen um die Partikelgröße zu messen, bis die Partikelgröße oder Qualität den Anforderungen entspricht, falls nicht, wird die Suspension in den Abfallbehälter 6 geführt. Die Suspensionen werden dann nach unterschiedlichen Methoden behandelt entsprechend der Wirkstoff Darreichungsform.

Zum Beispiel, falls die Verabreichungsform den Wirkstoff in Pulverform verlangt, wird die Nanopartikelsuspension in den Nachbehandlungsschritt 8 geführt, wie der Gefriertrocknung, Sprühtrocknung, Sprüh-Gefriertrocknung oder Fallfilmverdampfung um das Lösemittel zu entfernen und das getrocknete Nanopartikelpulver zu erhalten.

Falls die Zuführungsform eine Nanopartikelsuspension ist, wird die ursprüngliche Partikelsuspension weiter verdünnt oder aufkonzentriert, um die geeignete Konzentration an Wirkstoff in der Suspension zu erhalten. Optional ist Reinigungssolvent Feed 3 eingeplant, um die Wirkstoffpartikeln im Prozess aufzulösen, falls die Partikeln den Mikrokanalreaktor 5 verblocken oder die Nebenanlagenteile verblocken.

### Beispiel 1: Gefriertrocknung und Sprüh-Gefriertrocknung der gefällten Nanopartikelsuspension

Reinstwasser (Widerstandswert > 18,2 MOhm cm) wurde als Antisolvent im Prozess genutzt. Ethanol wurde als Solvent eingesetzt. Zur Förderung der Solvent und Antisolvent-Lösung wurden HPLC Pumpen mit konstantem Volumenstrom genutzt. Der Solvent-Feed wurde auf 20 °C temperiert und der Antisolvent Feed auf < 5 °C, sodass die Produkttemperatur bei < 5 °C lag. Im Solvent wurde Naproxen, als Wirkstoff und PVP K17 als Stabilisator gelöst und im Antisolvent 20 wt.-% Trehalose als Gerüstbildner. Die Wirkstoff- und Stabilisator-Konzentration lag bei 5 wt.-% und damit bei einer 1:1 Mischung. Der Antisolvent-Feed wurde bei 80 ml/min gefördert, während der Solvent-Feed bei 1 ml/min lag. Im Mischer wird eine Nanopartikelsuspension geformt. Die Partikelgröße wurde mittels Dynamischer Lichtstreuung (Malvern Nano Serie) ohne Filtration gemessen.

Die Nanosuspension wurde in einem Vergleichsversuch über einen Gefriertrocknungsschritt behandelt. Es entstand dabei ein fester, poröser Kuchen.

In einer erfindungsgemäßen Verarbeitung wurde die Nanosuspension mit einer Vertropfungsanlage vertropft und anschließend in einem Gefriertrocknungsschritt getrocknet. Es wurden Feststoffkugeln mit Partikelgrößen < 1,5 mm erhalten, die mit dem bloßen Auge erkennbar waren. Diese verfügten über eine ausgeprägte Fließ- und Schüttfähigkeit, welche vorteilhaft für die weitere Verarbeitung ist. Die redispergierten Nanopartikel wurden 60 min mit Ultraschall behandelt und zeigten einen Z-Average von 508 bzw. 618 nm.

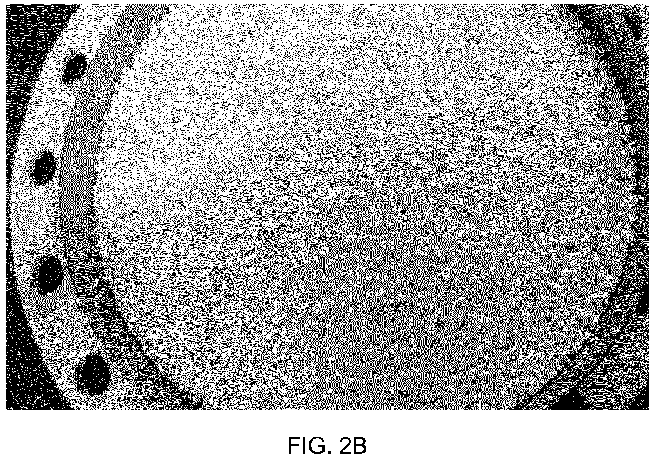

FIG. 2a zeigt die Nanopartikelsuspension mit 20 wt.-% Trehalose nach der Gefriertrocknung und FIG. 2B nach der Sprüh-Gefriertrocknung.

### Beispiel 2: Langzeitstabilität der redispergierten Nanopartikelsuspension

Die Nanosuspension wurde analog zu Beispiel 1 hergestellt. Im Solvent wurde Naproxen als Wirkstoff und PVP K30 als Stabilisator gelöst. Die Wirkstoffkonzentration lag bei 5 wt.-%, die Stabilisator-Konzentration lag bei 2,5 wt.-% und damit bei einer 2:1 Mischung. Der Antisolvent-Feed wurde bei 80 ml/min gefördert, während der Solvent-Feed bei 1 ml/min lag. Im Mischer wird eine Nanopartikelsuspension geformt. Die Partikelgröße wurde mittels dynamischer Lichtstreuung (Malvern Nano Serie) ohne Filtration gemessen. Der Gefriertrocknungsschritt erfolgte gemäß Beispiel 1.

Die Nanopartikelsuspension direkt nach der Fällung zeigte eine mittlere Partikelgröße von 66 nm und die redispergierte Nanopartikelsuspension nach 60 min Ultraschall-Behandlung wies eine Partikelgröße von 233 nm auf. Die redispergierte Nanopartikelsuspension hatte sich nach 3 Monaten kaum verändert und wies eine mittlere Partikelgröße von 265 nm auf. Die Partikelgrößenverteilung wurde nach 3 Monaten zusätzlich mit einem Laserbeuger (Mastersizer 3000) vermessen. Der D(90)-Wert lag dabei bei 260 nm.

**Tabelle 1: Langzeitstabilität der redispergierten Nanopartikelsuspension (Messung mittels Dynamischer Lichtstreuung)**

| | Mittlere Partikelgröße / nm | PDI |
|---|---|---|
| Nanopartikelsuspension direkt nach der Fällung | 62 | 0,218 |
| Redispergierte Nanopartikelsuspension | 233 | 0,15 |
| Redispergierte Nanopartikelsuspension nach > 3 Monaten | 265 | 0,116 |

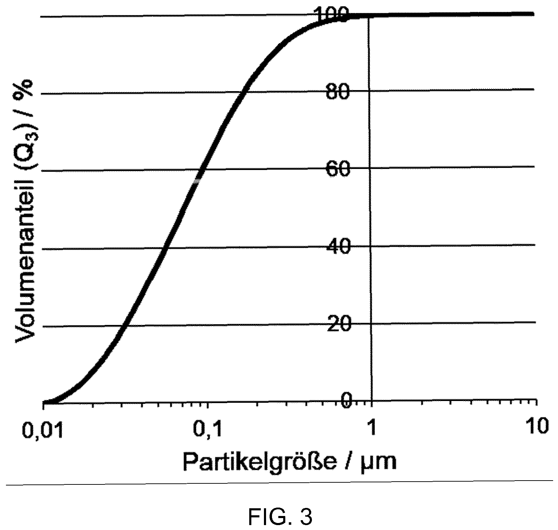

FIG. 3 zeigt die Partikelgrößenverteilung (Volumenverteilung) mittels Laserbeugung: D(10)-Wert 0,022 µm, D(50)-Wert 0,073 µm, D(90)-Wert 0,26 µm.

### Beispiel 3: Verbesserte Redispergierbarkeit durch Tensid-Zugabe

Die Nanosuspension wurde analog zu Beispiel 1 hergestellt. Im Solvent wurde Naproxen als Wirkstoff und PVP K17 als Stabilisator gelöst. Die Wirkstoff- und Stabilisatorkonzentration lag bei 5 wt.-% und damit bei einer 1:1 Mischung. Im Folgenden wurde im ersten Fall kein Tensid im Antisolvent verwendet. In einem zweiten Fall wurde das Tensid im Antisolvent, in diesem Fall SDS (Na-Dodecylsulfat), vorgelegt. Die Konzentration des SDS in Wasser lag bei 0,1 wt.-%. Der Antisolvent-Feed wurde bei 80 ml/min gefördert, während der Solvent-Feed bei 1 ml/min lag. Nach der Fällung wurde die Suspension entsprechend Beispiel 1 und 2 in einem Gefriertrocknungsschritt nachbehandelt und redispergiert. Im Gegensatz zu Beispiel 1 und 2 wurde kein Ultraschall zur Redispergierung verwendet.

Die Nanopartikelsuspension direkt nach der Fällung zeigte eine mittlere Partikelgröße von 66 nm auf. Nach der Redispergierung zeigt die Nanopartikelsuspension in der Laserbeugermessung ohne SDS einen D(90)-Wert von 58,9 µm und durch Zugabe von SDS 0,4 µm auf.

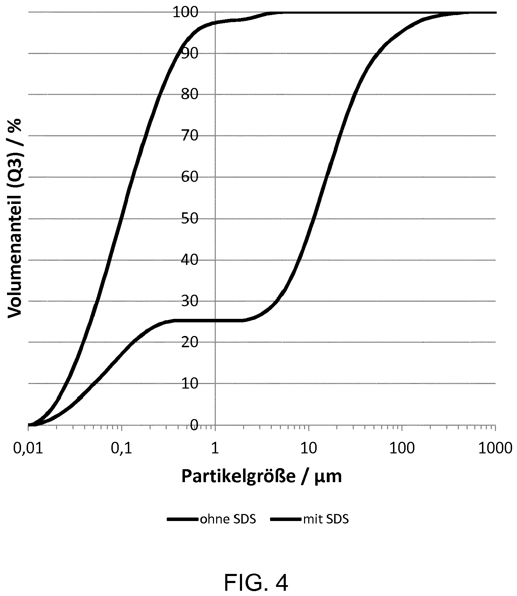

FIG. 4 zeigt die Partikelgrößenverteilung (Volumenverteilung) mittels Laserbeugung mit und ohne SDS-Zugabe.

## Patentansprüche

1. Verfahren zur Herstellung eines nanopartikulären Wirkstoffs, umfassend die Schritte:
a) Bereitstellen einer Lösung eines Wirkstoffs in einem Lösungsmittel, Bereitstellen eines Anti-Lösungsmittels für den Wirkstoff und Bereitstellen eines Stabilisators, wobei der Stabilisator in dem Lösungsmittel und/oder dem Anti-Lösungsmittel gelöst vorliegt und
b) Mischen der Lösung des Wirkstoffs in dem Lösungsmittel, des Antilösungsmittels und des Stabilisators in einem Mikromischer unter Erhalt einer Suspension umfassend ausgefällten Wirkstoff, das Lösungsmittel und das Anti-Lösungsmittel,
wobei der ausgefällte Wirkstoff in Form von Nanoteilchen vorliegt,
**dadurch gekennzeichnet, dass**
nach Schritt b) der Schritt c) durchgeführt wird:
c) Verdampfen des Lösungsmittels und des Anti-Lösungsmittels unter Erhalt von Aggregaten des nanopartikulären Wirkstoffs.

2. Verfahren gemäß Anspruch 1, wobei Schritt c) das Bereitstellen von Tröpfchen der Suspension beinhaltet und die Tröpfchen direkt nach ihrer Bereitstellung einen Durchmesser von ≥ 0.001 mm bis ≤ 3 mm aufweisen.

3. Verfahren gemäß Anspruch 1 oder 2, wobei die in Schritt b) ausgefallenen Nanoteilchen eine durchschnittliche Teilchengröße (bestimmt mittels Laserstreuung gemäß ISO 13320) von ≥ 20 to ≤ 300 nm aufweisen.

4. Verfahren gemäß einem der vorhergehenden Ansprüche, wobei Schritt c) ein Sprühtrocknungsschritt, ein Sprüh-Gefriertrocknungsschritt oder ein Gefriertrocknungsschritt ist.

5. Verfahren gemäß einem der vorhergehenden Ansprüche, wobei die erhaltenen Aggregate des nanopartikulären Wirkstoffs einen maximalen Durchmesser von ≥ 0.1 mm bis ≤ 3 mm aufweisen.

6. Verfahren gemäß einem der vorhergehenden Ansprüche, wobei der Volumenstrom des Solvents mit gelöstem Wirkstoff und der Volumenstrom des Antisolvents in einem Volumenverhältnis von Lösungsmittel zu Anti-Lösungsmittel ≥ 1:100 bis ≤ 1:1 zueinander liegen.

7. Verfahren gemäß einem der vorhergehenden Ansprüche, wobei in Schritt a) ein Tensid im Antilösungsmittel zugegeben wird.

8. Verfahren gemäß einem der vorhergehenden Ansprüche, wobei der Stabilisator im Lösungsmittel und/oder im Anti-Lösungsmittel gelöst vorliegt.

9. Verfahren gemäß einem der vorhergehenden Ansprüche, wobei der Mikromischer ein Kaskadenmischer oder ein Ventilmischer ist.

10. Verfahren gemäß einem der vorhergehenden Ansprüche, wobei nach Schritt b) die Suspension vom Mischer direkt in eine Sprühdüse eingeleitet wird und dort in Schritt c) versprüht und getrocknet wird.

11. Verfahren gemäß einem der vorhergehenden Ansprüche, wobei der Stabilisator einen Zucker, eine Aminosäure oder eine Mischung hieraus umfasst.

12. Verfahren gemäß einem der vorhergehenden Ansprüche, wobei der Stabilisator ein ionisches Tensid umfasst.

13. Verfahren gemäß einem der vorhergehenden Ansprüche, wobei der Stabilisator ein wasserlösliches Polymer umfasst.

14. Verfahren gemäß einem der vorhergehenden Ansprüche, wobei das Lösungsmittel ein Alkanol ist.

15. Verfahren gemäß einem der vorhergehenden Ansprüche, wobei das Anti-Lösungsmittel Wasser ist.
